# FASCICULE DE BREVET EUROPEEN

(11) **EP 0 973 032 B1**
(45) Date de publication et mention de la délivrance du brevet: **28.05.2008**
(21) Numéro de dépôt: 99401452.0
(22) Date de dépôt: 14.06.1999
(51) Int. Cl.: G01N 33/00

(54) **Système de prélevement de polluants spécifiques contenus dans des gaz d'échappement dilués issus de machines thermiques**
System zur Probenahme spezifischer Verunreinigungen, die sich in verdünnten Abgasen von thermischen Maschinen befinden
System for sampling specific pollutants in diluted thermal machine exhaust gases

(30) Priorité: 26.06.1998 FR 9808198
(43) Date de publication de la demande: 19.01.2000
(73) Titulaire: Institut Français du Pétrole, 92852 Rueil-Malmaison Cedex (FR)
(72) Inventeur: Pasquereau, Michel, 78500 Sartrouville (FR); Papagni, Jean-François, 78470 Saint Rémy les Chevreuses (FR); Levesque, Richard, 92150 Suresnes (FR); Dumas, Jean-Pierre, 78500 Sartrouville (FR); Montagne, Xavier, 92500 Rueil-Malmaison (FR)

(56) Documents cités:
- EP-A- 0 255 856
- DE-A- 19 607 574
- US-A- 4 758 521
- US-A- 4 759 210
- US-A- 5 279 146
- PATENT ABSTRACTS OF JAPAN vol. 012, no. 390 (P-772), 18 octobre 1988 (1988-10-18) & JP 63 132161 A (AGENCY OF IND SCIENCE & TECHNOL;OTHERS: 01), 4 juin 1988 (1988-06-04)
- PATENT ABSTRACTS OF JAPAN vol. 018, no. 250 (P-1736), 12 mai 1994 (1994-05-12) & JP 06 034500 A (HONDA MOTOR CO LTD), 8 février 1994 (1994-02-08)
- PATENT ABSTRACTS OF JAPAN vol. 016, no. 006 (C-0900), 9 janvier 1992 (1992-01-09) & JP 03 232516 A (NISSAN MOTOR CO LTD), 16 octobre 1991 (1991-10-16)

## Description

La présente invention concerne le domaine du prélèvement de fluides gazeux en vue de leur analyse, tels que les gaz d'échappement de machines thermiques à combustion.

Plus précisément, la présente invention concerne le prélèvement de fluides gazeux contenant des aldéhydes et cétones ainsi que des hydrocarbures.

Selon l'invention, ces deux modes de prélèvement peuvent être effectués simultanément ou non, en fonction d'un objectif analytique choisi.

Une utilisation avantageuse de l'invention a trait au prélèvement de gaz d'échappement de véhicules automobiles évoluant sur châssis dynamique au cours de cycles de conduites normalisés. La présente invention est conçue pour assurer automatiquement, au cours d'un cycle de conduite prédéterminé, plusieurs prélèvements associés à des phases précises de fonctionnement du véhicule.

A ce jour, la majorité des laboratoires d'analyse effectuant des mesures d'aldéhydes, cétones et hydrocarbures individuels dans les gaz d'échappement d'automobile sur banc à rouleaux travaillent avec leur propre matériel de mesure, sans liaison interactive entre ces différents éléments c'est-à-dire sans assurer véritablement un protocole de prélèvement fiable, précis et reproductible. Un exemple est JP 63 132 161, qui décrit un système d'analyse avec deux canaux, l'un pour les alcools et l'autre pour formaldéhyde.

Concernant les émissions d'aldéhydes et cétones, les cartouches de silice greffée à la dinitrophénylhydrazine qui permettent de prélever et dériver chimiquement en composés hydrazones ces espèces chimiques, sont actuellement les plus couramment utilisées. Chaque cartouche doit ensuite subir un traitement au laboratoire d'analyse pour dissoudre ces composés dans un solvant (acétonitrile). Cette technique de prélèvement possède l'avantage d'être efficace, simple d'utilisation et offre des possibilités de stockage à moyen terme des échantillons. Les dérivés hydrazones sont ensuite séparés en injectant un aliquote de cette solution dans un ensemble chromatographique CLHP (Chromatographie Liquide Haute Performance) et détectés individuellement en absorption UV (ultra violet).

En outre, il n'existe pas de matériel commercial compact utilisant de telles cartouches, qui assure les étapes indispensables à un protocole de prélèvement tel que le pilotage, le contrôle et l'enregistrement des paramètres de prélèvement essentiels à une quantification fiable et précise des émissions d'aldéhydes et cétones.

Concernant les émissions individuelles d'hydrocarbures en C1-C13, et afin de se placer dans des conditions similaires et normalisées des prélèvements des polluants classiques (CO, HC totaux, NOₓ), on utilise couramment des sacs de stockage en Tedlar (film polychlorure de vinyle chimiquement inerte et exempt de plastifiants). Généralement, ces sacs sont installés en parallèle aux installations existantes pour assurer une certaine représentativité de l'effluent dilué fourni au cours du cycle de fonctionnement.

D'autres méthodes connues réalisent après l'essai, un transfert des gaz recueillis dans les sacs de prélèvement vers de plus petits sacs (10 à 20 litres) pour les acheminer plus facilement vers le laboratoire d'analyse. Ensuite, un faible volume est injecté en chromatographie gazeuse suivant des protocoles analytiques particuliers (injection par boucle calibrée, reconcentration de l'échantillon sur supports solides...). Parfois on ne filtre pas les effluents gazeux émis par un véhicule diesel, ce qui pollue les sacs définitivement. Dans d'autres cas connus, la filtration est telle que des rétentions d'hydrocarbures peuvent être la cause de perte de composés hydrocarbonés notamment inférieurs au C13.

On ne connaît pas de matériel particulier relatif au prélèvement de polluants spécifiques tels que les aldéhydes, cétones, hydrocarbures permettant un échantillonnage selon des cycles de fonctionnement normalisés.

La présente invention permet ce type de prélèvements.

Elle permet notamment l'utilisation de cartouches connues, leur mise en place rapide et étanche dans un support de prélèvement adapté éliminant toute pollution extérieure, la programmation simple des paramètres d'échantillonnage, l'automaticité complète du prélèvement lorsque l'ordre est donné de démarrer le piégeage des effluents en fonction de la phase de fonctionnement du véhicule. De plus la présente invention permet un contrôle permanent des conditions de prélèvement au cours du cycle de fonctionnement (débits, températures, durée de piégeage).

En d'autres termes, la présente invention permet un prélèvement modulaire, automatisé et interactif des gaz émis par une machine thermique en fonctionnement.

Ainsi la présente invention a pour objet un système de prélèvement de polluants spécifiques contenus dans des gaz d'échappement dilués de machines thermiques à combustion comprenant un premier module dédié aux émissions d'aldéhydes et de cétones et un deuxième module destiné aux émissions d'hydrocarbures.

Selon l'invention, le système comprend en outre un moyen destiné à coordonner le ou lesdits prélèvements à des phases prédéterminées de fonctionnement de ladite machine thermique.

De façon particulière, le premier moyen comprend
- un circuit de piégeage des aldéhydes et cétones contenus dans les gaz d'échappement dilués comprenant une voie de simulation du passage des gaz disposée parallèlement à au moins une voie de prélèvement du gaz dilué, ces voies ayant une entrée commune pour les gaz dilués et une sortie commune reliée à une ligne comportant un élément filtrant, un régulateur de débit massique et une pompe disposés en série;
- un circuit de prélèvement de l'air de dilution comprenant une voie de simulation du passage des gaz disposée parallèlement à au moins une voie de prélèvement de l'air de dilution, ces voies ayant une entrée commune pour l'air de dilution et une sortie commune reliée à une ligne comportant un élément filtrant, un régulateur de débit massique et une pompe disposés en série.

Le premier module peut en outre comprendre un circuit de purge destiné à nettoyer les circuits ayant véhiculé les gaz dilués et l'air de dilution avant leur entrée dans le module de prélèvement lui-même.

Ainsi, le circuit de piégeage des aldéhydes et cétones comprend au moins une cartouche de prélèvement disposée dans une voie de prélèvement.

Une électrovanne peut être placée en aval de chaque cartouche de prélèvement, permettant soit le passage des gaz vers l'aval, soit l'obstruction de la voie correspondante.

De façon plus précise, le circuit de purge comprend une arrivée générale pour le gaz de purge, et deux voies parallèles l'une associée au circuit de piégeage des gaz d'échappement dilués, l'autre au circuit de piégeage de l'air de dilution, les deux voies débouchant par une extrémité sur l'arrivée générale du gaz de purge et par l'autre extrémité soit sur l'arrivée des gaz dilués, soit sur l'arrivée de l'air de dilution.

Avantageusement, chaque voie du circuit de purge comprend une électrovanne disposée près de l'arrivée générale du gaz de purge, permettant de couper la circulation dans la voie, et un élément destiné à fixer le débit du gaz de purge dans la voie.

Conformément à l'invention, le deuxième module de prélèvement des hydrocarbures comprend
- au moins une entrée générale pour les gaz d'échappement dilués, au moins une première voie équipée d'un sac de prélèvement, et une autre voie parallèle à la première voie et permettant la circulation des gaz en dehors dudit sac de prélèvement,
- un moyen d'orientation sélective des gaz d'échappement vers l'une ou l'autre desdites voies;
- une entrée générale pour l'air de dilution, au moins une voie équipée d'un sac de prélèvement et une autre voie permettant la circulation de l'air de dilution en dehors dudit sac de prélèvement.
- un moyen d'orientation sélective de l'air de dilution vers l'une ou l'autre desdites voies.

En outre, le deuxième moyen de traitement peut comprendre un circuit destiné à purger un ou plusieurs des sacs de prélèvement du gaz qu'il contient par cycle de vidange/remplissage.

Plus précisément, ledit circuit de purge comprend une entrée générale pour le gaz de purge, une première électrovanne reliée à une pompe, une deuxième électrovanne, une évacuation et un circuit de liaison vers les voies parallèles.

De façon particulière, la première électrovanne est reliée à l'entrée générale, à la pompe et au circuit de liaison vers les voies parallèles.

En outre, la deuxième électrovanne est reliée à l'évacuation générale, à la pompe et au circuit de liaison vers les voies parallèles.

Par ailleurs, le circuit de purge comprend en outre un filtre disposé entre la deuxième électrovanne et les voies parallèles associées aux sacs, ledit filtre étant destiné à épurer l'air lors des cycles de remplissage des gaz.

De façon spécifique, le moyen d'orientation des gaz d'échappement comprend une électrovanne placée sur la voie de circulation des gaz, une électrovanne sur la (ou chaque) voie équipée d'un sac de prélèvement et une électrovanne en amont desdites voies parallèles.

En outre, le moyen d'orientation de l'air de dilution comprend une électrovanne placée sur la voie de circulation de l'air, une électrovanne sur la (ou chaque) voie équipée d'un sac de prélèvement et une électrovanne en amont desdites voies parallèles.

Selon une particularité de l'invention, le deuxième module de prelèvement des hydrocarbures comprend deux voies parallèles au niveau de l'entrée générale des gaz dilués, l'une pour les gaz de moteurs diesel et l'autre pour les gaz de moteurs à allumage commandé.

De façon spécifique, la voie pour les gaz d'échappement diesel comprend un filtre chauffé et un élément destiné à fixer le débit massique du gaz à prélever, alors que l'autre voie comprend un élément pour fixer le débit massique du gaz à prélever et un filtre pour les impuretés.

En outre, le système comprend, en amont des voies parallèles pour le prélèvement des gaz dilués, une pompe et un débitmètre.

Par ailleurs, la ligne d'alimentation en air de dilution comprend, en amont des voies parallèles, un filtre, une pompe et un débitmètre réglable.

D'autres avantages, caractéristiques, détails de l'invention apparaîtront mieux à la lecture de la description qui va suivre faite à titre illustratif et nullement limitatif en référence aux dessins annexés sur lesquelles :
- La figure 1 est un schéma du moyen de prélèvement des aldéhydes et cétones;
- La figure 2 est un schéma du moyen de prélèvement des hydrocarbures; et
- La figure 3 est un schéma de l'unité de contrôle selon l'invention.

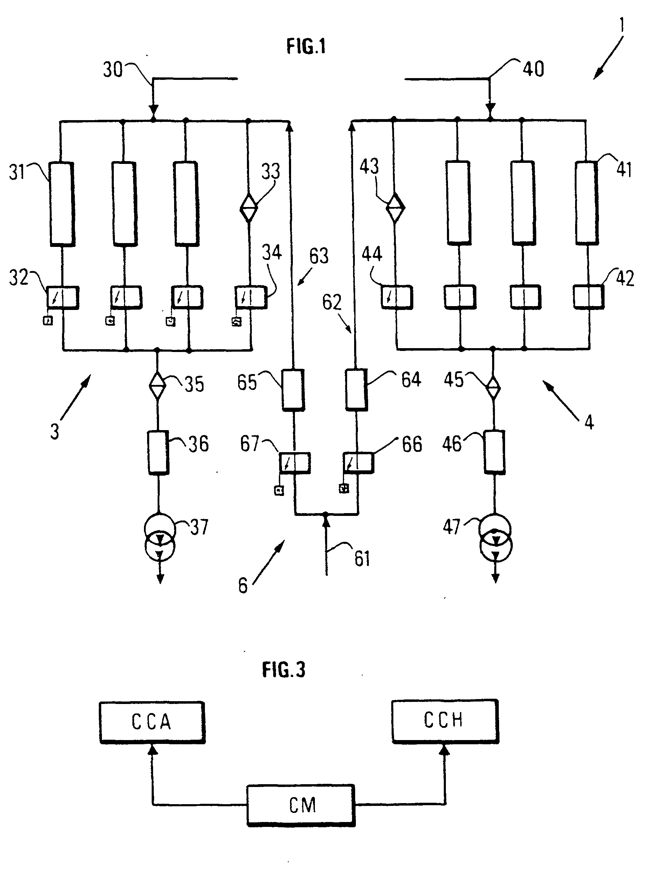

La figure 1 schématise le module 1 de prélèvement des aldéhydes et cétones selon l'invention.

Globalement, celui-ci comprend un circuit 3 de piégeage des aldéhydes et cétones constitué d'une entrée générale 30 pour lesdits gaz dilués, qui se sépare en plusieurs voies parallèles : les unes comprennent un moyen 31 de prélèvement des aldéhydes et cétones (tel qu'une cartouche waters Sep Pak Silica par exemple), et une électrovanne 32 pour arrêter sur commande l'écoulement dans chaque voie ; une autre voie parallèle est destinée à la simulation du passage des gaz dans les autres voies et elle comprend un filtre 33 et une électrovanne 34.

L'ensemble de ces voies parallèles présente une sortie commune sur une voie unique qui peut comprendre un filtre 35, un régulateur de débit massique 36 et une pompe 37 disposés en série.

Le premier module 1 comprend une deuxième entrée générale 40 d'un circuit 4 de prélèvement de l'air de dilution qui sert à diluer les gaz.

Le circuit 4 est organisé globalement comme le circuit 3 c'est-à-dire qu'il comprend plusieurs voies parallèles ayant l'entrée générale 40 en commun. Plusieurs voies (ici au nombre de trois) permettent le prélèvement de l'air de dilution, avec une cartouche 41 et une électrovanne 42 associée, en aval de la cartouche 41. Une autre voie permet la simulation du passage des gaz dans les voies de prélèvement ; cette voie comprend généralement un filtre 43 en série avec une électrovanne 44. Cette voie permet de faire circuler les gaz dilués lorsqu'il n'est pas jugé utile de les faire circuler dans les voies de prélèvement proprement dit. La commande des différentes électrovannes permet cette distribution sélective.

Tout moyen connu en soit peut être utilisé, sans sortir du cadre de l'invention, pour réaliser cette orientation (ou distribution) sélective des gaz. Ainsi une électrovanne multi-voie peut par exemple être utilisée.

La sortie commune (non référencée) des voies parallèles est reliée à l'entrée d'une voie unique qui comprend préférentiellement un filtre 45, un régulateur de débit massique 46 et une pompe 47.

Le module de prélèvement 1 assure donc un échantillonnage de l'air de dilution et des gaz dilués ce qui permet une détermination ultérieure de la composition des gaz non dilués.

Additionnellement le module 1 peut comprendre un circuit de purge 6 qui permet, une fois l'ensemble des prélèvements effectués au niveau du module 1, de purger à contre-courant les arrivées des gaz dilués et de l'air de dilution.

Un gaz de purge, de l'azote par exemple, est amené sur une arrivée générale 61 qui se sépare en deux conduits 62, 63 : l'un 62 débouchant vers l'arrivée 40 de l'air de dilution tandis que le second conduit 63 débouche à l'arrivée générale du gaz dilué 30.

Chaque conduit 62, 63 comprend une électrovanne 66, 67 et un moyen 64, 65 destiné à fixer le débit du gaz de purge ; ce moyen peut être un capillaire par exemple.

Préférentiellement l'air de dilution et les gaz dilués sont chauffés avant leur entrée dans le premier module, grâce à des cannes chauffantes par exemple.

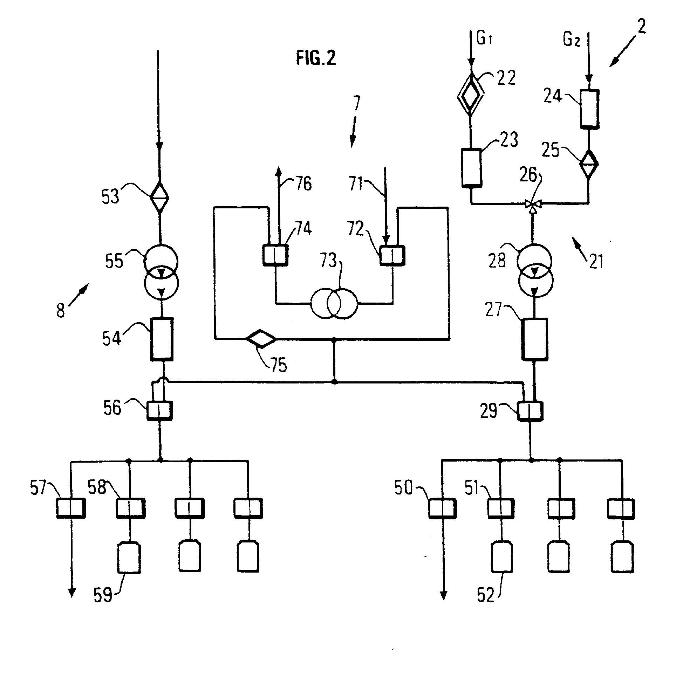

La figure 2 schématise les principaux éléments du module 2 de prélèvement des hydrocarbures qui comprend un premier ensemble 21 pour les gaz dilués et un deuxième ensemble 8 pour l'air de dilution. Additionnellement et non obligatoirement, un troisième ensemble 7 peut être envisagé, pour vidanger les éléments ayant été remplis de gaz.

De façon plus détaillée, l'ensemble 21 comprend au moins une entrée générale pour les gaz dilués. Selon le mode de réalisation de la figure 2, il existe deux entrées distinctes ; l'une G1 pour des gaz d'échappement de moteurs diesel et l'autre G2 pour des gaz d'échappement de moteurs à essence. Bien entendu ceci est tout à fait facultatif mais cela permet de traiter au mieux chaque type de gaz d'échappement de véhicule.

Dans ce contexte particulier, les gaz issus de G1 traversent un porte-filtre chauffé 22 puis un orifice calibré 23 ; le chauffage permet d'éviter une rétention des hydrocarbures ; les gaz issus de G2 traversent d'abord un orifice calibré 24 puis un filtre 25 destiné à retenir les grosses impuretés (agglomérats) éventuellement contenus dans les gaz d'échappement.

Les deux voies parallèles G1, G2 se rejoignent au niveau d'une vanne 26 à l'extrémité d'une voie de transfert équipée ici d'une pompe 28, d'un débitmètre 27, et d'une électrovanne 29.

La voie de transfert aboutit à une entrée commune pour plusieurs voies parallèles : ici trois voies parallèles sont prévues pour le prélèvement des hydrocarbures du gaz dilué ; une quatrième voie est aussi prévue pour une circulation de gaz avant tout prélèvement. La voie de circulation des gaz comprend une électrovanne 50 pour y régler le débit préalablement au prélèvement. Les trois voies parallèles sont chacune équipées d'une électrovanne 51 et d'un sac 52 destiné à recueillir les gaz.

Le nombre de voies équipées de sacs de prélèvement 52 n'est pas obligatoirement égal à trois. Une seule voie ou plus peuvent être envisagées sans sortir du cadre de l'invention.

Par ailleurs, les électrovannes 29, 50 et 51, qui apparaissent dans le mode de réalisation de l'invention illustré par la figure 2, peuvent être remplacées par tout moyen connu en soit, sans sortir du cadre de l'invention, pour répartir et orienter sélectivement les gaz dilués vers la voie de circulation ou l'une des voies équipées de sac 52.

Le deuxième ensemble 8 du module 2 de prélèvement des hydrocarbures concerne l'air de dilution qui arrive sur un filtre 53 puis sur une pompe 55 reliée à une électrovanne 56 et sur un débitmètre réglable 54; tous ces éléments sont en série sur la ligne de dilution qui aboutit sur une entrée commune à plusieurs voies parallèles. L'une des voies comprend uniquement une électrovanne 57 et elle est destinée à faire passer l'air de dilution avant ou après la phase de prélèvement proprement dite.

Les autres voies parallèles, ici au nombre de trois, comportent chacune une électrovanne 58 et un sac de prélèvement 59.

Sans sortir du cadre de l'invention, les électrovannes 56, 57 et 58 peuvent être remplacées par tout autre moyen permettant d'orienter sélectivement l'air de dilution vers la voie de circulation ou vers l'une des voies équipées de sacs 59.

Par ailleurs, le module 2 peut comprendre un système 7 de vidange et de remplissage des sacs de prélèvement 52, 59. En mode évacuation, les sacs seront vidés de l'air extérieur qui les a rempli préalablement (en phase de remplissage). Ce mode opératoire, facultatif, permet de réutiliser les mêmes sacs pour plusieurs campagnes de prélèvement.

Le système 7 comprend une ligne 71 d'alimentation en air extérieur qui, en mode de remplissage, traverse une première électrovanne 72 une pompe 73, une deuxième électrovanne 74 puis un filtre 75 qui permet d'épurer l'air lors des cycles de rinçage. L'air extérieur est ensuite séparé en deux : une partie est dirigée vers l'électrovanne 56 côté air de dilution si les sacs 59 doivent être nettoyés ; une autre partie de l'air extérieur est dirigée vers l'électrovanne 29 si les sacs 52 de prélèvement des gaz dilués doivent être nettoyés. Chaque électrovanne 29, 56 commandée par un système central, autorise ou non le passage de l'air extérieur.

En mode évacuation, l'air contenu dans les sacs en est évacué en faisant le cheminement suivant : il passe dans la première électrovanne 12 puis dans la pompe 73 et dans la deuxième électrovanne 74, pour sortir finalement par une ligne spécifique 76.

L'ensemble qui vient d'être décrit comprend en outre un pilotage des différentes électrovannes et autres éléments actifs, afin que le ou les prélèvements soient réalisés en fonction par exemple de la phase de fonctionnement du véhicule qui émet des gaz d'échappement.

Une unité de contrôle telle que schématisée sur la figure 3 est prévue à cet effet. Cette unité de gestion des commandes est constituée d'un automate programmable permettant de mémoriser l'ensemble des paramètres de prélèvement au cours du cycle de fonctionnement, d'assurer le pilotage interactif avec les installations du banc à rouleaux et d'activer par rapport à sa programmation initiale les différents éléments constituant les circuits de prélèvement (pompes, électrovannes...).

Le moyen CM représente le dispositif qui assure le contrôle de la machine thermique, avec notamment l'ensemble des paramètres caractérisant une phase de son fonctionnement.

Le moyen CM envoie des signaux de commande appropriés à un moyen CCA de contrôle du premier module 1, ainsi que des signaux à un moyen CCH de contrôle du deuxième module 2.

Il est ainsi possible selon l'invention de programmer des séquences d'échantillonnage par rapport à des cycles de fonctionnement prédéterminés, par exemple par rapport à des cycles de conduite normalisés.

A la fin de la procédure de prélèvement, les échantillons stockés sur les supports solides (type cartouches 31, 41) ou dans des sacs de prélèvement 52, 59 sont amenés dans un laboratoire d'analyse où leur analyse chromatographique permet la quantification des aldéhydes présents à la fois dans l'air de dilution et dans le gaz dilué, d'où il s'en suit une détermination de la quantité de polluants dans des gaz non dilués.

## Revendications

1. Système de prélèvement de polluants spécifiques contenus dans des gaz d'échappement dilués issus de machines thermiques à combustion comprenant un premier module (1) dédié aux émissions d'aldéhydes et de cétones et un deuxième module (2) destiné à prélever les émissions d'hydrocarbures, **caractérisé en ce qu'**il comprend en outre un moyen destiné à coordonner le ou lesdits prélèvements à des phases prédéterminées de fonctionnement de ladite machine thermique.

2. Système de prélèvement et de mesure selon la revendication 1, **caractérisé en ce que** le premier module (1) comprend :
- un circuit (3) de piégeage des aldéhydes et cétones contenus dans les gaz d'échappement dilués comprenant une voie de simulation du passage des gaz disposée parallèlement à au moins une voie de prélèvement du gaz dilué, ces voies ayant une entrée commune (30) pour les gaz dilués et une sortie commune reliée à une ligne comportant un élément filtrant (35), un régulateur de débit massique (36) et une pompe (37) disposés en série;
- un circuit (4) de prélèvement de l'air de dilution comprenant une voie de simulation du passage des gaz disposée parallèlement à au moins une voie de prélèvement de l'air de dilution, ces voies ayant une entrée commune (40) pour l'air de dilution et une sortie commune reliée à une ligne comportant un élément filtrant (45), un régulateur de débit massique (46) et une pompe (47) disposés en série.

3. Système selon la revendication 2, **caractérisé en ce que** le premier module (1) comprend en outre un circuit de purge (6) destiné à nettoyer les circuits ayant véhiculé les gaz dilués et l'air de dilution avant leur entrée dans le module (1) de prélèvement lui-même.

4. Système selon l'une quelconque des revendications précédentes, **caractérisé en ce que** le circuit de piégeage des aldéhydes et cétones comprend au moins une cartouche de prélèvement (31) disposée dans une voie de prélèvement.

5. Système selon la revendication 4, **caractérisé en ce qu'**une électrovanne (32) est placée en aval de chaque cartouche de prélèvement (31), permettant soit le passage des gaz vers l'aval, soit l'obstruction de la voie correspondante.

6. Système selon l'une quelconque des revendications 3 à 5, **caractérisé en ce que** le circuit de purge (6) comprend une arrivée générale (61) pour le gaz de purge, et deux voies parallèles (62, 63) l'une associée au circuit (3) de piégeage des gaz d'échappement dilués, l'autre au circuit (4) de piégeage de l'air de dilution, les deux voies débouchant par une extrémité sur l'arrivée générale (61) du gaz de purge et par l'autre extrémité soit sur l'arrivée (30) des gaz dilués, soit sur l'arrivée (40) de l'air de dilution.

7. Système selon la revendication 6, **caractérisé en ce que** chaque voie (62, 63) du circuit de purge (6) comprend une électrovanne (66, 67) disposée près de l'arrivée générale (61) du gaz de purge, permettant de couper la circulation dans la voie, et un élément (64, 65) destiné à fixer le débit du gaz de purge dans la voie (62, 63).

8. Système selon l'une quelconque des revendications précédentes, **caractérisé en ce que** le deuxième module (2), destiné au prélèvement des hydrocarbures comprend :
- au moins une entrée générale pour les gaz d'échappement dilués (G1. G2), au moins une première voie équipée d'un sac de prélèvement (52), et une autre voie parallèle à la première voie et permettant la circulation des gaz en dehors dudit sac de prélèvement,
- un moyen d'orientation sélective des gaz d'échappement vers l'une ou l'autre desdites voies;
- une entrée générale pour l'air de dilution, au moins une voie équipée d'un sac de prélèvement (59) et une autre voie permettant la circulation de l'air de dilution en dehors dudit sac de prélèvement.
- un moyen d'orientation sélective de l'air de dilution vers l'une ou l'autre desdites voies.

9. Système selon la revendication 8, **caractérisé en ce que** le deuxième moyen de traitement comprend en outre un circuit (7) destiné à purger un ou plusieurs des sacs de prélèvement (52, 59) du gaz qu'il contient par cycle de vidange/remplissage.

10. Système selon la revendication 9, **caractérisé en ce que** ledit circuit de purge (7) comprend une entrée générale (71) pour le gaz de purge, une première électrovanne (72) reliée à une pompe (73), une deuxième électrovanne (74), une évacuation (76) et un circuit de liaison vers les voies parallèles.

11. Système selon la revendication 10, **caractérisé en ce que** la première électrovanne (72) est reliée à l'entrée générale (71), à la pompe (73) et au circuit de liaison vers les voies parallèles.

12. Système selon l'une quelconque des revendications 10 ou 11, **caractérisé en ce que** la deuxième électrovanne (74) est reliée à l'évacuation générale (76), à la pompe (73) et au circuit de liaison vers les voies parallèles.

13. Système selon l'une quelconque des revendications 9 à 12, **caractérisé en ce que** le circuit de purge (7) comprend en outre un filtre (75) disposé entre la deuxième électrovanne (74) et les voies parallèles associées aux sacs, ledit filtre (75) étant destiné à épurer l'air lors des cycles de remplissage des sacs.

14. Système selon l'une quelconque des revendications 8 à 13, **caractérisé en ce que** le moyen d'orientation des gaz d'échappement comprend une électrovanne (50) placée sur la voie de circulation des gaz, une électrovanne (51) sur la (ou chaque) voie équipée d'un sac de prélèvement et une électrovanne (29) en amont desdites voies parallèles.

15. Système selon l'une quelconque des revendications 8 à 14, **caractérisé en ce que** le moyen d'orientation de l'air de dilution comprend une électrovanne (57) placée sur la voie de circulation de l'air, une électrovanne (58) sur la (ou chaque) voie équipée d'un sac de prélèvement et une électrovanne (56) en amont desdites voies parallèles.

16. Système selon l'une quelconque des revendications 8 à 15, **caractérisé en ce que** le deuxième module (2) de prélèvement des hydrocarbures comprend deux voies parallèles (G1, G2) au niveau de l'entrée générale des gaz dilués, l'une (G1) pour les gaz de moteurs diesel et l'autre (G2) pour les gaz de moteurs à allumage commandé.

17. Système selon la revendication 16, **caractérisé en ce que** la voie pour les gaz d'échappement diesel comprend un filtre chauffé (22) et un élément (23) destiné à fixer le débit massique du gaz à prélever.

18. Système selon l'une quelconque des revendications 16 ou 17, **caractérisé en ce que** la voie pour les gaz d'échappement de moteurs à allumage commandé comprend un élément (24) destiné à fixer le débit massique du gaz à prélever et un filtre (25) pour les impuretés.

19. Système selon l'une quelconque des revendications 16 à 18, **caractérisé en ce qu'**il comprend en outre en amont des voies parallèles pour le prélèvement des gaz dilués, une pompe (28) et un débitmètre (27).

20. Système selon l'une quelconque des revendications 8 à 19, **caractérisé en ce que** la ligne d'alimentation en air de dilution comprend, en amont des voies parallèles, un filtre (53), une pompe (55) et un débitmètre réglable (54).

## Claims

1. A system for drawing off specific pollutants contained in diluted exhaust gases coming from combustion heat engines including a first module (1) dedicated to aldehyde and ketone emissions and a second module (2) for drawing off hydrocarbon emissions, **characterised in that** it further includes a means for coordinating the draw-off(s) at predetermined operational phases of said heat engine.

2. The drawing-off and measuring system according to Claim 1, **characterised in that** the first module (1) includes:
- a circuit (3) for trapping the aldehydes and ketones contained in the diluted exhaust gases including a path for simulating the passage of the gases arranged parallel to at least one path for drawing off the diluted gas, whereby these paths have a common entrance (30) for the diluted gases and a common exit connected to a line comprising a filtering element (35), a mass flow regulator (36) and a pump (37) arranged in series;
- a circuit (4) for drawing off the dilution air including a path for simulating the passage of the gases arranged parallel to at least one path for drawing off dilution air, whereby these paths have a common entrance (40) for the dilution air and a common exit connected to a line comprising a filtering element (45), a mass flow regulator (46) and a pump (47) arranged in series.

3. The system according to Claim 2, **characterised in that** the first module (1) further includes a purging circuit (6) for cleaning the circuits which have transported the diluted gases and the dilution air before they enter into the drawing-off module (1) itself.

4. The system according to any one of the preceding claims, **characterised in that** the circuit for trapping the aldehydes and ketones includes at least one drawing-off cartridge (31) arranged in a drawing-off path.

5. The system according to Claim 4, **characterised in that** a solenoid valve (32) is positioned downstream of each drawing-off cartridge (31), enabling either the passage of the gases in a downstream direction or the obstruction of the corresponding path.

6. The system according to any one of Claims 3 to 5, **characterised in that** the purging circuit (6) includes a general entrance (61) for the purging gas, and two parallel paths (62, 63), whereby one is associated with the circuit (3) for trapping the diluted exhaust gases and the other is associated with the circuit (4) for trapping the dilution air, whereby the two paths open out at one end into the general entrance (61) for the purging gas and at the other end either into the entrance (30) for the diluted gases or into the entrance (40) for the dilution air.

7. The system according to Claim 6, **characterised in that** each path (62, 63) of the purging circuit (6) includes a solenoid valve (66, 67) arranged near the general entrance (61) for the purging gas, enabling the circulation within the path to be stopped, and an element (64, 65) for setting the purging gas flow in the path (62, 63).

8. The system according to any one of the preceding claims, **characterised in that** the second module (2) for drawing off the hydrocarbons includes:
- at least one general entrance for the diluted exhaust gases (G1, G2), at least one first path equipped with a drawing-off bag (52), and another path which is parallel to the first and which enables the circulation of the gases outside said drawing-off bag,
- a means for selectively orientating the exhaust gases towards one or the other of said paths;
- a general entrance for the dilution air, at least one path equipped with a drawing-off bag (59) and another path enabling the circulation of the dilution air outside said drawing-off bag,
- a means for selectively orientating the dilution air towards one or the other of said paths.

9. A system according to Claim 8, **characterised in that** the second treatment means further includes a circuit (7) for purging one or more drawing-off bags (52, 59) of the gas it contains by means of an emptying/filling cycle.

10. The system according to Claim 9, **characterised in that** said purging circuit (7) includes a general entrance (71) for the purging gas, a first solenoid valve (72) connected to a pump (73), a second solenoid valve (74), an evacuation (76) and a connection circuit to the parallel paths.

11. The system according to Claim 10, **characterised in that** the first solenoid valve (72) is connected to the general entrance (71), to the pump (73) and to the connection circuit to the parallel paths.

12. The system according to any one of Claims 10 or 11, **characterised in that** the second solenoid valve (74) is connected to the general evacuation (76), to the pump (73) and to the connection circuit to the parallel paths.

13. The system according to any one of Claims 9 to 12, **characterised in that** the purging circuit (7) further includes a filter (75) arranged between the second solenoid valve (74) and the parallel paths associated with the bags, said filter (75) being intended for purifying the air during the bag filling cycles.

14. The system according to any one of Claims 8 to 13, **characterised in that** the means for orientating the exhaust gases includes a solenoid valve (50) positioned on the gas circulation path, a solenoid valve (51) on the (or each) path equipped with a drawing-off bag and a solenoid valve (29) upstream of said parallel paths.

15. The system according to any one of Claims 8 to 14, **characterised in that** the means for orientating the dilution air includes a solenoid valve (57) positioned on the air circulation path, a solenoid valve (58) on the (or each) path equipped with a drawing-off bag and a solenoid valve (56) upstream of said parallel paths.

16. The system according to any one of Claims 8 to 15, **characterised in that** the second module (2) for drawing off the hydrocarbons includes two parallel paths (G1, G2) at the general entrance for the diluted gases, one (G1) for the gases of diesel engines and the other (G2) for the gases of controlled ignition engines.

17. The system according to Claim 16, **characterised in that** the path for the diesel exhaust gases includes a heated filter (22) and an element (23) for setting the mass flow of the gas to be drawn off.

18. The system according to any one of Claims 16 or 17, **characterised in that** the path for the exhaust gases of controlled ignition engines includes an element (24) for setting the mass flow of the gas to be drawn off and a filter (25) for impurities.

19. The system according to any one of Claims 16 to 18, **characterised in that** it further includes, upstream of the parallel paths for drawing off the diluted gases, a pump (28) and a flowmeter (27).

20. The system according to any one of Claims 8 to 19, **characterised in that** the dilution air supply line includes, upstream of the parallel paths, a filter (53), a pump (55) and an adjustable flowmeter (54).

## Patentansprüche

1. System zur Entnahme von spezifischen Schadstoffen, die in verdünnten Abgasen enthalten sind, die aus Verbrennungskraftmaschinen kommen, umfassend eine erste Einheit (1) für Aldehyd- und Ketonemissionen und eine zweite Einheit (2) zur Entnahme von Kohlenwasserstoffemissionen, **dadurch gekennzeichnet, dass** es außerdem ein Mittel umfasst, um die Entnahme(n) in vorherbestimmten Betriebsphasen der Kraftmaschine zu koordinieren.

2. Entnahme- und Messsystem nach Anspruch 1, **dadurch gekennzeichnet, dass** die erste Einheit (1) folgendes umfasst:
- einen Kreislauf (3) zum Einfangen der Aldehyde und Ketonen, die in den verdünnten Abgasen enthalten sind, umfassend einen Kanal zum Simulieren des Gasdurchgangs, der parallel zu mindestens einem Kanal zur Entnahme des verdünnten Gases angeordnet ist, wobei diese Kanäle einen gemeinsamen Eingang (30) für die verdünnten Gase und einen gemeinsamen Ausgang haben, der mit einer Leitung verbunden ist, die ein Filterelement (35), einen Massenstromregler (36) und eine Pumpe (37), die in Reihe geschaltet sind, umfasst;
- einen Kreislauf (4) zur Entnahme der Verdünnungsluft, umfassend einen Kanal zum Simulieren des Gasdurchgangs, der parallel zu mindestens einem Kanal zur Entnahme der Verdünnungsluft angeordnet ist, wobei diese Kanäle einen gemeinsamen Eingang (40) für die Verdünnungsluft und einen gemeinsamen Ausgang haben, der mit einer Leitung verbunden ist, die ein Filterelement (45), einen Massenstromregler (46) und eine Pumpe (47), die in Reihe geschaltet sind, umfasst.

3. System nach Anspruch 2, **dadurch gekennzeichnet, dass** die erste Einheit (1) außerdem einen Entlüftungskreislauf (6) umfasst, der dazu gedacht ist, die Kreisläufe zu reinigen, welche die verdünnten Gase und die Verdünnungsluft vor ihrem Eintritt in die Entnahmeeinheit (1) selber transportiert haben.

4. System nach einem der vorhergehenden Ansprüche, **dadurch gekennzeichnet, dass** der Kreislauf zum Einfangen der Aldehyde und Ketonen mindestens einen Entnahmeeinsatz (31) umfasst, der in einem Entnahmekanal angeordnet ist.

5. System nach Anspruch 4, **dadurch gekennzeichnet, dass** ein Magnetventil (32) unterhalb jedes Entnahmeeinsatzes (31) eingesetzt ist, das entweder den Durchgang der Gase nach unten oder die Versperrung des entsprechenden Kanals ermöglicht.

6. System nach einem der Ansprüche 3 bis 5, **dadurch gekennzeichnet, dass** der Entlüftungskreislauf (6) eine Hauptzuführung (61) für das Entlüftungsgas und zwei Parallelkanäle (62, 63) umfasst, wobei der eine zum Kreislauf (3) zum Einfangen der verdünnten Entlüftungsgase und der andere zum Kreislauf (4) zum Einfangen der Verdünnungsluft gehört, wobei die beiden Kanäle über das eine Ende an der Hauptzuführung (61) des Entlüftungsgases und über das andere Ende entweder an der Zuführung (30) der verdünnten Gase oder an der Zuführung (40) der Verdünnungsluft ausmünden.

7. System nach Anspruch 6, **dadurch gekennzeichnet, dass** jeder Kanal (62, 63) des Entlüftungskreislaufs (6) ein Magnetventil (66, 67), das in der Nähe der Hauptzuführung (61) des Entlüftungsgases angeordnet ist, wodurch der Umlauf im Kanal abgeschaltet werden kann, und ein Element (64, 65), das dazu gedacht ist, den Durchfluss des Entlüftungsgases im Kanal (62, 63) festzusetzen, umfasst.

8. System nach einem der vorhergehenden Ansprüche, **dadurch gekennzeichnet, dass** die zweite Einheit (2), die zur Kohlenwasserstoffentnahme gedacht ist, folgendes umfasst:
- mindestens einen Haupteingang für die verdünnten Abgase (G1, G2), mindestens einen ersten Kanal, der mit einem Entnahmebeutel (52) ausgestattet ist, und einen anderen Kanal, der zum ersten Kanal parallel ist und den Umlauf der Gase außerhalb des Entnahmebeutels ermöglicht;
- ein Mittel zur wahlweisen Orientierung der Abgase auf den einen oder anderen Kanal;
- einen Haupteingang für die Verdünnungsluft, mindestens einen Kanal, der mit einem Entnahmebeutel (59) ausgestattet ist, und einen anderen Kanal, der den Umlauf von Verdünnungsluft außerhalb des Entnahmebeutels ermöglicht;
- ein Mittel zur wahlweisen Orientierung der Verdünnungsluft zum einen oder anderen Kanal.

9. System nach Anspruch 8, **dadurch gekennzeichnet, dass** das zweite Verarbeitungsmittel außerdem einen Kreislauf (7) umfasst, der dazu gedacht ist, einen oder mehrere der Entnahmebeutel (52, 59) des Gases, das er enthält, pro Entleerungs-/Füllungszyklus zu entlüften.

10. System nach Anspruch 9, **dadurch gekennzeichnet, dass** der Entlüftungskreislauf (7) einen Haupteintritt (71) für das Entlüftungsgas, ein erstes Magnetventil (72), das mit einer Pumpe (73) verbunden ist, ein zweites Magnetventil (74), einen Ablass (76) und einen Verbindungskreislauf zu den Parallelkanälen umfasst.

11. System nach Anspruch 10, **dadurch gekennzeichnet, dass** das erste Magnetventil (72) mit dem Haupteingang (71), der Pumpe (73) und dem Verbindungskreislauf zu den Parallelkanälen verbunden ist.

12. System nach einem der Ansprüche 10 oder 11, **dadurch gekennzeichnet, dass** das zweite Magnetventil (74) mit dem Hauptablass (76), der Pumpe (73) und dem Verbindungskreislauf zu den Parallelkanälen verbunden ist.

13. System nach einem der Ansprüche 9 bis 12, **dadurch gekennzeichnet, dass** der Entlüftungskreislauf (7) außerdem einen Filter (75) umfasst, der zwischen dem zweiten Magnetventil (74) und den zu den Beuteln gehörenden Parallelkanälen angeordnet ist, wobei der Filter (75) dazu gedacht ist, die Luft Während der Befüllungszyklen der Beutel zu säubern.

14. System nach einem der Ansprüche 8 bis 13, **dadurch gekennzeichnet, dass** das Mittel zum Orientieren der Abgase ein Magnetventil (50), das in den Gasumlaufkanal gesetzt ist, und ein Magnetventil (51) in dem (bzw. jedem) Kanal, der mit einem Entnahmebeutel ausgestattet ist, und ein Magnetventil (29) oberhalb der Parallelkanäle umfasst.

15. System nach einem der Ansprüche 8 bis 14, **dadurch gekennzeichnet, dass** das Mittel zum Orientieren der Verdünnungsluft ein Magnetventil (57), das in den Luftumlaufkanal gesetzt ist, ein Magnetventil (58) in dem (bzw. jedem) Kanal, der mit einem Entnahmebeutel ausgestattet ist, und ein Magnetventil (56) oberhalb der Parallelkanäle umfasst.

16. System nach einem der Ansprüche 8 bis 15, **dadurch gekennzeichnet, dass** die zweite Einheit (2) zur Entnahme der Kohlenwasserstoffe zwei Parallelkanäle (G1, G2) am Haupteingang der verdünnten Gase umfasst, wobei der eine (G1) für die Gase von Dieselmotoren und der andere (G2) für die Gase von Motoren mit Funkenzündung bestimmt ist.

17. System nach Anspruch 16, **dadurch gekennzeichnet, dass** der Kanal für die Dieselabgase einen beheizten Filter (22) und ein Element (23), das dazu gedacht ist, den Massenstrom des zu entnehmenden Gases festzulegen, umfasst.

18. System nach einem der Ansprüche 16 oder 17, **dadurch gekennzeichnet, dass** der Kanal für die Abgase von Motoren mit Funkenzündung ein Element (24), das dazu gedacht ist, den Massenstrom des zu entnehmenden Gases festzulegen, und einen Filter (25) für die Verunreinigungen umfasst.

19. System nach einem der Ansprüche 16 bis 18, **dadurch gekennzeichnet, dass** es außerdem oberhalb der Parallelkanäle für die Entnahme der verdünnten Gase eine Pumpe (28) und einen Durchflussmesser (27) umfasst.

20. System nach einem der Ansprüche 8 bis 19, **dadurch gekennzeichnet, dass** die Leitung zur Versorgung mit Verdünnungsluft oberhalb der Parallelkanäle einen Filter (53), eine Pumpe (55) und einen einstellbaren Durchflussmesser (54) umfasst.
